(19) Europäisches Patentamt / European Patent Office / Office européen des brevets

(11) **EP 4 144 324 B1**

(12) **EUROPEAN PATENT SPECIFICATION**

(45) Date of publication and mention
of the grant of the patent:
**28.08.2024 Bulletin 2024/35**

(21) Application number: **21796451.9**

(22) Date of filing: **21.04.2021**

(51) International Patent Classification (IPC):
**A61C 13/00** (2006.01)  **A61C 9/00** (2006.01)
**G16H 50/50** (2018.01)

(52) Cooperative Patent Classification (CPC):
**A61C 13/0004; A61C 9/004; G16H 50/50**

(86) International application number:
**PCT/CN2021/088569**

(87) International publication number:
**WO 2021/218724 (04.11.2021 Gazette 2021/44)**

(54) **INTELLIGENT DESIGN METHOD FOR DIGITAL MODEL FOR ORAL DIGITAL IMPRESSION INSTRUMENT**

INTELLIGENTES ENTWURFSVERFAHREN FÜR EIN DIGITALES MODELL FÜR EIN ORALES DIGITALES ABDRUCKINSTRUMENT

PROCÉDÉ DE CONCEPTION INTELLIGENTE POUR MODÈLE NUMÉRIQUE POUR INSTRUMENT D'IMPRESSION NUMÉRIQUE ORAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.04.2020 CN 202010360675**

(43) Date of publication of application:
**08.03.2023 Bulletin 2023/10**

(73) Proprietor: **Chengdu Besmile Medical Technology
Corporation
Limited
Chengdu, Sichuan 610041 (CN)**

(72) Inventors:
• **WANG, Changjian**
  **Chengdu, Sichuan 610000 (CN)**
• **ZHANG, Dengkai**
  **Chengdu, Sichuan 610000 (CN)**
• **YAN, Xinzhang**
  **Chengdu, Sichuan 610000 (CN)**
• **LU, Guanghui**
  **Chengdu, Sichuan 610000 (CN)**
• **CAI, Hongbin**
  **Chengdu, Sichuan 610000 (CN)**

(74) Representative: **Elzaburu S.L.P.
Edificio Torre de Cristal
Paseo de la Castellana 259 C, planta 28
28046 Madrid (ES)**

(56) References cited:
EP-A1- 2 422 740      CN-A- 101 548 911
CN-A- 106 504 331     CN-A- 107 019 570
CN-A- 107 230 255     CN-A- 110 025 387
CN-A- 111 568 592     JP-A- 2000 107 203
JP-A- 2000 107 203    US-A1- 2015 073 577

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to the technical field of medical computer aided design, and particularly to an intelligent design method of a digital model for an oral digital impression instrument.

BACKGROUND

When producing dental implants in the oral cavity or oral rehabilitation traditionally, dentists usually use intraoral scanners to scan the patient's oral cavity to obtain the 3D model data of the teeth in the oral cavity, and then transmit them to professional design factories for design and processing. Professional design factories also need to design manually according to the design requirements proposed by dentists and their own design experience; however, this traditional semi-manual design method is time-consuming, inefficient, and has a long production period. In addition, the designers in the factory do not directly contact the patients, so it is difficult to adjust the standard teeth to the optimal size and pattern. As a result, the fabricated teeth cannot be suitable for the patients. After installation, dentists need to make subsequent adjustments.

Related technologies are known from JP2000107203A, US2015/073577A1 and EP2422740A1, the above-mentioned applications all disclose a method for digitally designing a dental crown.

JP2000107203A disclose a model to deform effectively by registering the models in which a tool axis is inclined at a prescribed angle in a database and adjusting the tooth axis angle of the selected model based on measured shape data concerning the deformation. Models are grouped for the kind of a tooth and stored in accordance with various specifications in a database and in each model, the tooth axis is previously set at a prescribed standard angle. A model processing part forms a surface model to be the peripheral information of a tooth deficiency part from three-dimensional coordinate value measurement data which are obtained by a measuring means and a margin extracting means extracts a margin line from the surface model of an abutment tooth. A selecting means compares and collates the surface model with the model constituting a crown shape and selects the desired model from the database. An articulation simulation means deforms the selected model so as to be adapted at a correlative position against the surface model.

US2015/073577A1 disclose a method of obtaining orientation and localization of at least one dental implant includes the following steps: (a) obtaining an impression comprising at least one fixated impression abutment corresponding to the at least one dental implant(s) and/or obtaining an impression comprising at least one fixated impression abutment corresponding to the at least one dental implant(s) on which is mounted a scan implant/analog (b) obtaining pre-determined information of the shape of the impression abutment and/or scan implant/analog, (c) scanning at least a part of the impression, wherein the part includes the at least one impression abutment and/or scan implant/analog thereby obtaining scan data, (d) determining the orientation and localization of the dental implant based on the pre-determined information and the scan data.

EP2422740A1 discloses a computer-implemented method for digitally designing a dental restoration for a rest tooth, wherein the rest tooth is described by data of the rest tooth and wherein a tooth template is described by data of the tooth template, by means of an Laplacian surface deformation to deform the tooth template towards the surface of the rest tooth, wherein the method uses, for the Laplacian surface deformation, an angle weighting function depending on: the angle between the normal direction of a target on the surface of the rest tooth or the normal direction of a handle on the tooth template; and the direction of a line connecting the target and the handle; wherein the angle weighting function is used to weight the handle for deforming the tooth template towards the rest tooth. Further, the invention is related to a computer-readable medium having stored thereon instructions, which when executed by a processor, are adapted to perform the method steps of the inventive computer-implemented method.

SUMMARY

In order to solve the above problem, the present invention provides an intelligent design method of a digital model for an oral digital impression instrument. The present application is set out in the appended set of claims.

In order to achieve the above purpose, the technical solution adopted in the present invention is as defined in the appended claims.

The method comprises the following steps :

1) database establishment: tooth data being stored in an xml file, and stored alternative dental crown data information including a tooth number, a dental crown type as a single crown or crown bridge, an overall size feature vector of a dental crown, and a storage path of a tooth;

2) data acquisition: acquiring 3D model data of an oral cavity model after tooth preparation with oral digital acquisition equipment, the data acquired by the oral digital acquisition equipment including an upper jaw and a lower jaw, as well as confirming occluding relations between the upper jaw and the lower jaw;

3) data preprocessing: labeling the 3D model of the oral cavity obtained in step 2), and labeling an abutment tooth position, single crown/crown bridge and an abutment edge line;

4) intelligent design: performing automatic design processing on a to-be-produced tooth according to the 3D model obtained by the preprocessing in step 3), and matching several alternative dental crowns from the database;

5) alternative dental crown matching: determining the similarity between the alternative dental crown and the to-be-produced tooth according to the cosine of an included angle between the overall size feature vector of the dental crown of the alternative dental crown and the overall size feature vector of the dental crown of the to-be-produced tooth, the cosine of the included angle being [0, 1];

6) dental pattern adjustment: adjusting according to the scaling of the overall size feature vector of the to-be-produced tooth, a maxillofacial feature vector and the alternative dental crown;

7) data output: the designed tooth model being exported as a data format which is configured to imported by 3shape and EXOcad software.

According to the invention, the overall size feature vector of the dental crown in step 1) is ($size\_x, size\_y, size\_z$), which is obtained through calculation by a six-point method, and the six-point method obtains six outline high points through the maximum and minimum tooth crown coordinates, of which two outline high points of the mesial and distal surfaces are $P_{min}^x$ and $P_{max}^x$, two outline high points of the buccal and lingual surfaces are $P_{min}^y$ and $P_{max}^y$, and two outline high points of the bottom and top surfaces are $P_{min}^z$ and $P_{max}^z$; $size\_x$ is the absolute value of the difference between x coordinates of the outline high points $P_{min}^x$ and $P_{max}^x$, $size\_y$ is the absolute value of the difference between y coordinates of the outline high points $P_{min}^y$ and $P_{max}^y$, and $size\_z$ is the absolute value of the difference between z coordinates of the outline high points $P_{min}^z$ and $P_{max}^z$.

Data acquisition in step 2) comprises the following steps:

1.1) acquiring 3D model data of the upper jaw and the lower jaw and buccal data at occlusion respectively;

1.2) calculating and fixing the contact relation between the 3D models of the upper jaw and the lower jaw through buccal data, or manually translating and rotating the 3D models of the upper jaw and the lower jaw in the 3D coordinate system, so that the 3D models of the upper jaw and the lower jaw are in a correct occluding relation.

Data preprocessing in step 3) comprises the following steps:

2.1) selecting the abutment after tooth preparation, highlighting it, and recording the tooth number of the tooth position, the tooth number rule of the tooth position being given by the FDI notation;

2.2) selecting the type of the abutment as a single crown or a crown bridge: if it is a single crown, recording the tooth number of the abutment; if it is a crown bridge, recording the tooth numbers of the leftmost and rightmost abutments of the crown bridge and the tooth numbers of all to-be-produced teeth;

2.3) marking the edge lines interactively, selecting the points of triangular mesh on the edge line of the abutment, sketching the points of the abutment edge line one by one, and completing the selection of the abutment edge line; and highlighting the final edge line with a special color.

the intelligent design in step 4) comprises the following steps:

3.1) calculating the distance between adjacent teeth: interactively selecting the two points closest to the adjacent teeth on the abutment edge line, and calculating the distance between these two points to obtain the distance between adjacent teeth;

3.2) calculating the buccal and lingual dental arch convexity curves: fitting the dental arch convexity curve by a β function-based method according to the 3D model data of the upper jaw and the lower jaw obtained in step 2);

3.3) calculating the occlusal gum diameter height: calculating the distance from each point on the abutment edge line to the occlusal plane according to the selected abutment edge line, and calculating the average distance as the occlusal gum diameter height;

3.4) calculating the cusp pit and fissure ridge shape features of opposite jaws: calculating the occlusal surface features of the opposite jaw tooth according to the 3D model data of the upper jaw and lower jaw obtained in step 2).

Step 3.2) comprises the following steps:

3.2.1) obtaining the buccal apex of incisor contact surface of the jaw where the abutment is, the point of bilateral canines with the maximum buccal curvature, and the point of bilateral second permanent molars with the maximum buccal curvature;

3.2.2) determining the $\beta$ function according to the five points obtained in step 3.2.1) to fit a dental arch convexity curve;

3.2.3) repeating steps 3.2.1) and 3.2.2), and calculating the lingual dental arch convexity curve in the same manner.

Step 3.4) comprises the following steps:

3.4.1) interactively selecting the occlusal surface of the opposite jaw tooth;

3.4.2) calculating the feature vector of the selected occlusal surface: first, obtaining the effective neighborhood around the cusp pit and fissure ridge in the occlusal surface according to the selection results, then establishing a local spherical coordinate system for the surface, next, calculating the elevation and azimuth of the normal at each vertex of the surface through the 2D histogram statistical method, and determining the position index in turn; finally, generating the maxillofacial feature vector ($f1$, $f2$,..., $fn$) of the opposite jaw tooth according to the 2D histogram.

According to the invention, the alternative dental crown matching in step 5) comprises the following steps:

4.1) tooth number and tooth type matching: finding out alternative dental crown data in the database according to the tooth number of the selected abutment and the dental crown type selected as single crown or crown bridge;

4.2) overall size matching: calculating the overall size feature vector of the dental crown of the to-be-produced tooth as ($size \_ x_0, size \_ y_0, size \_ z_0$) and the overall size feature vector of the dental crown of the alternative dental crown as ($size \_ x', size \_ y', size \_ z'$), and determining the similarity between the alternative dental crown and the to-be-produced tooth according to the cosine between the overall size feature vector of the dental crown of the alternative dental crown and the overall size feature vector of the dental crown of the to-be-produced tooth; the larger the cosine of the included angle, the smaller the included angle of the two vectors; when the directions of the two vectors coincide, the maximum cosine of the included angle is 1;

4.3) local feature matching: calculating the complementary vector ($f1'$, $f2'$,..., $fn'$) of the maxillofacial feature vector ($f1$, $f2$,..., $fn$) of the opposite jaw tooth of the to-be-produced tooth, the complementary vector ($f1'$, $f2'$,..., $fn'$) = (-$f1$,-$f2$,...,-$fn$), and conducting local feature matching through calculating the cosine similarity of the vector ($f1'$, $f2'$,..., $fn'$) and the maxillofacial feature vector ($F1$, $F2$,..., $Fn$) of the alternative dental crown.

The dental pattern adjustment in step 6) comprises the following steps:

5.1) integral adjustment: adjusting by virtue of scaling matrix S according to the overall size feature vector of the to-be-produced tooth and the scaling of the alternative dental crown, in order to ensure the best size matching of the alternative dental crown;

5.2) occlusal surface adjustment: adjusting the normal vector of the dental surface of the alternative dental crown according to the maxillofacial feature vector of the opposite jaw tooth of the to-be-produced tooth to best fit;

5.3) adjusting the lower edge of the dental crown, adjusting the coordinates of each point on the lower edge of the alternative dental crown according to the distance from each point on the abutment edge line to the occlusal plane calculated in step 3.3), so that the distance from the each point on the lower edge of the alternative dental crown to the highest point on the occlusal surface matches the distance from each point on the abutment edge line to the occlusal plane.

The intelligent design method of the digital oral model is adapted for use beside or in proximity to a dental chair.
The intelligent design method of a digital model for an oral digital impression instrument has the following advantages that: through computer aided design, several matching alternative dental crowns are quickly retrieved from the database, and the most suitable alternative dental crowns are determined by the similarity of the overall size feature vectors of the to-be-produced tooth and the alternative dental crown. The design method produces to-be-produced teeth with high precision and efficiency, accelerate the denture processing speed, reduce the intermediate links of processing, and change the clinical work process and communication between doctors and patients.

BRIEF DESCRIPTION OF THE DRAWINGS

FIG. 1 is the flow chart of an intelligent design technology for a digital oral model of the present invention;

FIG. 2 shows five determined points to fit the dental arch convexity curve;

FIG. 3 shows the calculation method of e value in $\beta$ function;

FIG. 4 shows 6 outline high points of the tooth model.

DETAILED DESCRIPTION

The present invention belongs to the technical field of medical computer aided design technology, and particularly to an intelligent design method of a digital model for an oral digital impression instrument. Based on oral digital acquisition equipment, such as oral digital impression instrument, the present invention provides an intelligent tooth design technology, which can intelligently generate teeth by calculating various quantitative data after tooth preparation in a virtual 3D environment.

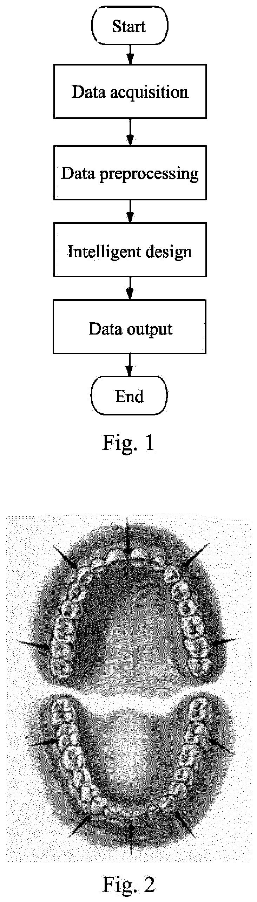

The flow chart as shown in Fig. 1 illustrates the specific process of the entire implementation of the present invention:

1) Data acquisition: acquiring 3D model data of an oral cavity model after tooth preparation with oral digital acquisition equipment, the data acquired by the oral digital acquisition equipment including an upper jaw and a lower jaw, as well as confirming occluding relations between the upper jaw and the lower jaw.
This step comprises the following steps:

1.1) acquiring 3D model data of the upper jaw and the lower jaw and buccal data at occlusion, respectively; and

1.2) calculating and fixing the contact relation between the 3D model of the upper (lower) jaw through buccal data, or manually translating and rotating the 3D model of the lower (upper) jaw in the 3D coordinate system, so that the 3D models of the upper jaw and the lower jaw are in a correct occluding relation.

2) Data preprocessing: first labeling the 3D model of the oral cavity obtained in step 2), and labeling an abutment tooth position, single crown/crown bridge and an abutment edge line.
This step comprises the following steps:

2.1) selecting the abutment after tooth preparation, highlighting it, and recording the tooth number of the tooth position(s), the tooth numbering rule of the tooth position(s) being given or defined by the FDI notation;

2.2) selecting the type of the abutment (single crown or crown bridge): if it is a single crown, recording the tooth number of the abutment; if it is a crown bridge, recording the tooth numbers of the leftmost and rightmost abutments of the crown bridge and the tooth numbers of all to-be-produced teeth; and

2.3) marking the edge lines interactively: clicking on or selecting the points of triangular mesh on the edge line of the abutment with a mouse, sketching the points of the abutment edge line one by one, and if necessary completing the selection of the abutment edge line; during the sketching process, the 3D model of the jaw is configured to translate or rotate to avoid incomplete occlusion; and highlighting the final edge line, for example with a special color.

3) Intelligent design: automatically designing the produced teeth according to the 3D model data obtained in step 2.
This step comprises the following steps:

3.1) calculating the distance between adjacent teeth: interactively selecting the two points closest to the adjacent teeth on the abutment edge line, and calculating the distance between these two points to obtain the distance between adjacent teeth, the specific calculation formula being shown as formula (1):

$$d = \sqrt{(x_2 - x_1)^2 + (y_2 - y_1)^2 + (z_2 - z_1)^2} \qquad \text{Formula (1)}$$

where, $(x_2, y_2, z_2)$, $(x_1, y_1, z_1)$ are the 3D coordinates of two points on the abutment; and

3.2) calculating the buccal and lingual dental arch convexity curves: fitting the dental arch convexity curve by a $\beta$ function-based method according to the 3D model data of the upper jaw and the lower jaw obtained in step 1).

This step comprises the following steps:

3.2.1) obtaining the buccal apex of incisor contact surface of the jaw where the abutment is, the point of bilateral canines with the maximum buccal curvature, and the point of bilateral second permanent molars with the maximum buccal curvature, with the five points as shown in Fig. 2;
3.2.2) determining $\beta$ function according to the five points obtained in step 3.2.1) to fit the dental arch convexity curve. The formula of $\beta$ function is shown as formula (2):

$$Y = D\left[1 - (2X/W)^2\right]^{e} \qquad \text{Formula (2)}$$

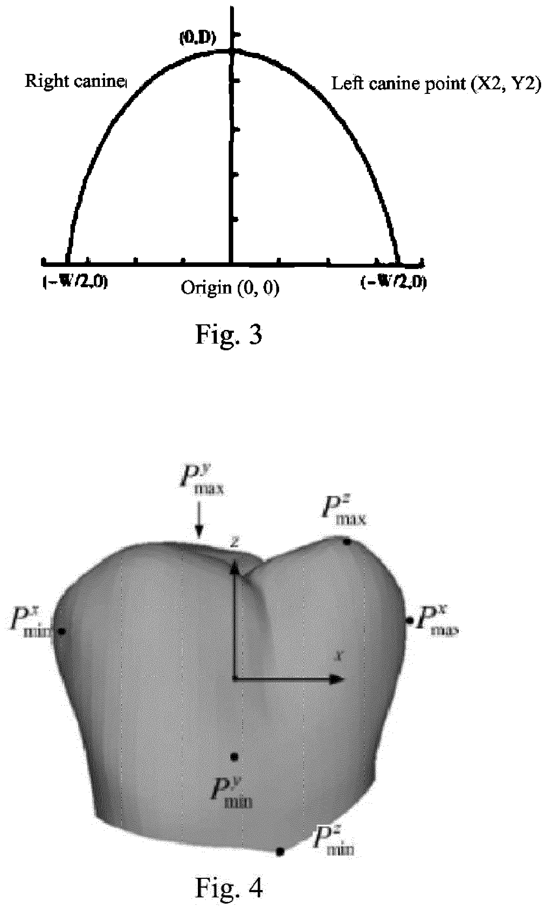

where: D is the width of the second permanent molar, that is, the distance between the points of bilateral second permanent molars with the maximum buccal curvature; W is the depth of the second permanent molar, that is, the distance between the connecting lines from the buccal apex of incisor contact surface to the point of bilateral second permanent molars with the maximum buccal curvature; e is determined by the position of canine, so that the distance from the curve to the point of bilateral canines with the maximum buccal curvature is the shortest and equal; the schematic diagram for calculating e value is as shown in Fig. 3. Once D, W and e are calculated, the buccal dental arch convexity curve is determined;
3.2.3) repeating steps 3.2.1) and 3.2.2), and calculating the lingual dental arch convexity curve in the same manner.

Step 3) above comprises furthermore the following steps:

3.3) calculating the occlusal gum diameter height, calculating the distance from each point on the edge line to the occlusal plane according to the selected abutment edge line, and calculating the average distance as the occlusal gum diameter height, the specific calculation formula(s) being shown as Formulas (3) and (4):

$$d_i = \left| \frac{Ax_i + By_i + Cz_i + D}{\sqrt{A^2 + B^2 + C^2}} \right| \qquad \text{Formula (3)}$$

where the equation of the occlusal plane is $Ax + By + Cz + D = 0$, and the coordinate of the point on the abutment edge line is $(x_i, y_i, z_i)$;

$$d = \frac{1}{n} \sum_{i=1}^{n} d_i \qquad \text{Formula (4)}$$

where $d$ is the calculated average distance, $d_i$ is the distance from a point on the abutment edge line to the occlusal plane, and $n$ is the number of points on the abutment edge line; and
3.4) calculating the cusp pit and fissure ridge shape features of opposite jaws: calculating the occlusal surface features of the opposite jaw teeth according to the 3D model data of the upper jaw and lower jaw obtained in step 1).

This step comprises the following steps:

> 3.4.1) interactively selecting the occlusal surface of the opposite jaw tooth; and
> 3.4.2) calculating the selected dental surface feature vector: first, obtaining the effective neighborhood around the cusp pit and fissure ridge in the occlusal surface according to the selection results; then establishing a local spherical coordinate system for the surface; next, calculating the elevation and azimuth of the normal at each vertex of the surface through the 2D histogram statistical method, and determining the position index in turn; finally, generating the maxillofacial feature vector ($f1$, $f2$,..., $fn$) of the opposite jaw tooth according to the 2D histogram.

Step 3) above comprises furthermore the following step:
3.5) data matching: matching the data of the to-be-produced tooth obtained previously with the data of the alternative dental crown in the database to quickly obtain a quasi-dental crown or bridge.
3.5.1) database establishment.
To facilitate the management of a standard tooth database, xml files is used to store teeth, including tooth number, tooth type (single crown/crown bridge), tooth feature vector, and tooth storage path.
Six outline high points are obtained by calculating the coordinates of the tooth model. Two of the outline high points $P^x_{min}$ and $P^x_{max}$ are on the mesial and distal surfaces, two of the outline high points $P^y_{min}$ and $P^y_{max}$ are on the buccal and lingual surfaces, and two of the outline high points $P^z_{min}$ and $P^z_{max}$ are on the bottom and top surfaces, as shown in Fig. 4.

The coordinates of the six outline high points are recorded as $P^x_{min}(x_1, y_1, z_1)$, $P^x_{max}(x_2, y_2, z_2)$, $P^y_{min}(x_3, y_3, z_3)$, $P^y_{max}(x_4, y_4, z_4)$, $P^z_{min}(x_5, y_5, z_5)$, $P^z_{max}(x_6, y_6, z_6)$; the absolute value of the difference between x coordinates of the outline high points $P^x_{min}$ and $P^x_{max}$ is taken as the mesio-distal dimension, recorded as *size _ x*; the absolute value of the difference between y coordinates of the outline high points $P^y_{min}$ and $P^y_{max}$ is taken as the buccolingual diameter, recorded as *size _ y*; the absolute value of the difference between z coordinates of the outline high points $P^z_{min}$ and $P^z_{max}$ is used to limit the jaw-gum diameter, recorded as *size _ z*; thus the calculation formula is shown as formula (5):

$$size\_x = |x_2 - x_1|$$

$$size\_y = |y_4 - y_3| \qquad \text{Formula (5)}$$

$$size\_z = |z_6 - z_5|$$

The overall size feature vector (*size _ x,size _ y,size _ z*) of the alternative dental crown is obtained.
In the meantime, the cusp pit and fissure ridge feature vector ($F1,F2,...,Fn$) of the alternative dental crown is calculated by reference to 3.4.2), that is, the maxillofacial feature vector of the alternative dental crown.
Step 3.5) above comprises furthermore:

> 3.5.2) tooth number and tooth type matching: finding alternative dental crown data in the database according to the tooth number of the selected abutment and the selected dental crown type (single crown or crown bridge);

> 3.5.3) overall size matching: obtaining the feature vector (*size _ $x_0$,size _ $y_0$,size _ $z_0$*) composed of three size parameters of the to-be-produced tooth from step 3.1) - step 3.4); the specific calculation method is shown as formula (5), and the overall size feature vector of the dental crown of the alternative dental crown is calculated from step 3.1) - step 3.4) as (*size _ x',size _ y',size _ z'*). The cosine of the included angle between the two vectors is calculated to measure the similarity between the feature vector of the to-be-produced tooth and the feature vectors of the alternative dental crown, and the cosine range of the included angle is [0,1]; the larger the cosine of the included angle, the smaller the included angle of the two vectors. When the directions of the two vectors coincide, the maximum cosine of the included angle is 1. The specific calculation formula of the cosine of the included angle is shown as formula (6):

$$\cos(\theta) = \frac{a \cdot b}{\|a\| \times \|b\|} = \frac{\sum_{i=1}^{n}(x_i \times y_i)}{\sqrt{\sum_{i=1}^{n}(x_i)^2} \times \sqrt{\sum_{i=1}^{n}(y_i)^2}} \qquad \text{Formula (6)};$$

and

3.5.4) local feature matching: calculating the complementary vector ($f1'$, $f2'$,..., $fn'$) of the maxillofacial feature vector ($f1$, $f2$,..., $fn$) of the opposite jaw tooth of the to-be-produced tooth, where ($f1'$, $f2'$,..., $fn'$) = ($-f1$,$-f2$,...,$fn$), and conducting local feature matching through calculating the cosine similarity of the vector ($f1'$, $f2'$,..., $fn'$) and the cusp pit and fissure ridge feature vector ($F1$, $F2$,..., $Fn$) of the alternative dental crown.

Step 3) above comprise furthermore:

3.6) dental pattern adjustment: since the matched data should not fully meet the requirements, adjusting the tooth position according to the calculation results of 3.1), 3.2) and 3.3);

3.6.1) integral adjustment: the three size parameters of the to-be-produced tooth are $size\_x_0$, $size\_y_0$ and $size\_z_0$, and the three size parameters of the alternative dental crown are $size\_x'$, $size\_y'$ and $size\_z'$. Suppose the scaling of the alternative dental crown in x, y and z directions as $s_x$, $s_y$ and $s_z$, the basic form of the homogeneous coordinate transformation matrix for scaling is as shown in formula (7):

$$S = \begin{bmatrix} s_x & 0 & 0 & 0 \\ 0 & s_y & 0 & 0 \\ 0 & 0 & s_z & 0 \\ 0 & 0 & 0 & 1 \end{bmatrix} \qquad \text{Formula (7)}$$

where the scaling is $s_x$, $s_y$ and $s_z$ respectively, obtained by the following formula (8):

$$s_x = size\_x_0 / size\_x'$$

$$s_y = size\_y_0 / size\_y' \qquad \text{Formula (8)}$$

$$s_z = size\_z_0 / size\_z'$$

The alternative dental crown is scaled by virtue of the scaling matrix S, so that the size of the alternative dental crown is best fit.

3.6.2) occlusal surface adjustment: adjusting the normal vector of the occlusal surface of the alternative dental crown according to the maxillofacial feature vector of the opposite jaw tooth of the to-be-produced tooth to best fit;

3.6.3) adjusting the lower edge of the dental crown: adjusting the coordinates of each point on the lower edge of the dental crown according to the distance from each point on the abutment edge line to the occlusal plane calculated in step 3.3), so that the distance from each point on the lower edge of the dental crown to the highest point on the occlusal surface matches the distance from each point on the abutment edge line to the occlusal plane.

4) Data output: the designed tooth model being exported as a data format which is imported by 3 shape and EXOcad software.

The rapid intelligent design method of the present invention is combined with the intraoral scanners and applied in the chair side design. When dentists finish the intraoral scan with the intraoral scanners, they can directly perform real-time design rapidly near the chair. Patients can observe the denture models immediately, thus improving the patients' experience. The data acquisition, data preprocessing and intelligent design steps can also be integrated into the intraoral scanner. After the intraoral scanner completes a preliminary design, the data is transmitted to the design computer beside the dental chair. The dentists can carry out secondary manual design according to the actual inspection, and then conduct quick matching in the database to accelerate the denture design.

**Claims**

1. An intelligent design method of a digital model for an oral digital impression instrument, wherein the method comprising:

   1) database establishment: alternative dental crown data being stored in an xml file, at least one of stored alternative dental crown data information including a tooth number, a dental crown type, an overall size feature vector of a dental crown, and a storage path of a tooth;
   the overall size feature vector of the dental crown is ($size\_x, size\_y, size\_z$), which is obtained through calculation by a six-point method, and the six-point method is to obtain six outline high points through the maximum and minimum tooth crown coordinates, of which, the two outline high points of the mesial and distal surfaces are $P_{min}^{x}$ and $P_{max}^{x}$, the two outline high points of the buccal and lingual surfaces are $P_{min}^{y}$ and $P_{max}^{y}$, and the two outline high points of the bottom and top surfaces are $P_{min}^{z}$ and $P_{max}^{z}$; $size\_x$ is the absolute value of the difference between x coordinates of the outline high points $P_{min}^{x}$ and $P_{max}^{x}$, $size\_y$ is the absolute value of the difference between y coordinates of the outline high points $P_{min}^{y}$ and $P_{max}^{y}$, and $size\_z$ is the absolute value of the difference between z coordinates of the outline high points $P_{min}^{z}$ and $P_{max}^{z}$;
   2) data acquisition: acquiring 3D model data of an oral cavity model after tooth preparation with oral digital acquisition equipment, the data acquired by the oral digital acquisition equipment including an upper jaw and a lower jaw, as well as confirming occluding relations between the upper jaw and the lower jaw;
   3) data preprocessing: labeling the 3D model of the oral cavity obtained in step 2), and labeling an abutment tooth position, the dental crown type and an abutment edge line;
   4) intelligent design: performing automatic design processing on a to-be-produced tooth according to the 3D model obtained by the preprocessing in step 3), and matching one of alternative dental crowns from the database;
   5) alternative dental crown matching: determining the similarity between an alternative dental crown and a to-be-produced tooth according to the cosine of an included angle between the overall size feature vector of the dental crown of the alternative dental crown and the overall size feature vector of the dental crown of the to-be-produced tooth, the cosine of the included angle being [0,1];
   the alternative dental crown matching in step 5) comprises furthermore:

      4.1) tooth number and dental crown type matching: finding out alternative dental crown data in the database according to the tooth number of the selected abutment and the dental crown type selected as single crown or crown bridge;
      4.2) overall size matching: calculating the overall size feature vector of the dental crown of the to-be-produced tooth as ($size\_x_0, size\_y_0, size\_z_0$) and the overall size feature vector of the dental crown of the alternative dental crown as ($size\_x', size\_y', size\_z'$), and determining the similarity between the alternative dental crown and the to-be-produced tooth according to the cosine between the overall size feature vector of the dental crown of the alternative dental crown and the overall size feature vector of the dental crown of the to-be-produced tooth; the larger the cosine of the included angle, the smaller the included angle of the two vectors; when the directions of the two vectors coincide, the maximum cosine of the included angle is 1;
      4.3) local feature matching: calculating the complementary vector ($f1', f2',..., fn'$) of the occlusal surface feature vector ($f1, f2,..., fn$) of the opposite jaw tooth of the to-be-produced tooth, the complementary vector

(*f1'*, *f2'*,..., *fn'*) = (-*f1*,-*f2*,...,-*fn*), and conducting local feature matching through calculating the cosine similarity of the complementary vector (*f1'*, *f2'*,..., *fn'*) and the maxillofacial feature vector (*F1*, *F2*,..., *Fn*) of the alternative dental crown;

6) dental pattern adjustment: adjusting according to a scaling of the overall size feature vector of the to-be-produced tooth, a maxillofacial feature vector and the alternative dental crown;

7) data output: the designed tooth model being exported as a data format which is adapted to be imported by 3shape and EXOcad software.

2. The intelligent design method according to claim 1, wherein data acquisition in step 2) comprises furthermore:

1.1) acquiring 3D model data of the upper jaw and the lower jaw and buccal data at occlusion respectively;
1.2) calculating and fixing the contact relation between the 3D models of the upper jaw and the lower jaw through buccal data, or manually translating and rotating the 3D models of the upper jaw and the lower jaw in the 3D coordinate system, so that the 3D models of the upper jaw and the lower jaw are in a correct occluding relation.

3. The intelligent design method according to claim 1, wherein data preprocessing in step 3) furthermore:

2.1) selecting the abutment after tooth preparation, highlighting it, and recording the tooth number of the tooth position, the tooth number rule of the tooth position being given by the FDI notation;
2.2) selecting the dental crown type of the abutment as a single crown or a crown bridge: if it is a single crown, recording the tooth number of the abutment; if it is a crown bridge, recording the tooth numbers of the leftmost and rightmost abutments of the crown bridge and the tooth numbers of all to-be-produced teeth;
2.3) marking the edge lines interactively: selecting the points of triangular mesh on the edge line of the abutment, sketching the points of the abutment edge line one by one, and completing the selection of the abutment edge line; highlighting the final edge line with a special color.

4. The intelligent design method according to claim 1, wherein intelligent design in step 4) comprises furthermore:

3.1) calculating the distance between adjacent teeth: interactively selecting the two points closest to the adjacent teeth on the abutment edge line, and calculating the distance between these two points to obtain the distance between adjacent teeth;
3.2) calculating the buccal and lingual dental arch convexity curves: fitting the dental arch convexity curve by the β function based method according to the 3D model data of the upper jaw and the lower jaw obtained in step 2);
3.3) calculating the occlusal gum diameter height: calculating the distance from each point on the abutment edge line to the occlusal plane according to the selected abutment edge line, and calculating the average distance as the occlusal gum diameter height;
3.4) calculating the cusp pit and fissure ridge shape features of opposite jaws: calculating the occlusal surface features of the opposite jaw teeth according to the 3D model data of the upper jaw and lower jaw obtained in step 2).

5. The intelligent design method according to claim 4, wherein step 3.2) comprises furthermore:

3.2.1) obtaining the buccal apex of incisor contact surface of the jaw where the abutment is, the point of bilateral canines with the maximum buccal curvature, and the point of bilateral second permanent molars with the maximum buccal curvature;
3.2.2) determining β function according to the five points obtained in step
3.2.1) to fit the dental arch convexity curve;
3.2.3) repeating steps 3.2.1) and 3.2.2), and calculating the lingual dental arch convexity curve in the same manner.

6. The intelligent design method according to claim 4, wherein step 3.4) comprises furthermore:

3.4.1) interactively selecting the occlusal surface of the opposite jaw tooth;
3.4.2) calculating the feature vector of the selected occlusal surface: first, obtaining the effective neighborhood around the cusp pit and fissure ridge in the occlusal surface according to the selection results, then establishing a local spherical coordinate system for the surface, next, calculating the elevation and azimuth of the normal at each vertex of the surface through the 2D histogram statistical method, and determining the position index in turn; finally, generating the maxillofacial feature vector ($f1$, $f2$,..., $fn$) of the opposite jaw tooth according to the 2D histogram.

7. The intelligent design method according to claim 4, wherein dental pattern adjustment in step 6) comprises furthermore:

5.1) integral adjustment: adjusting by virtue of scaling matrix S according to the overall size feature vector of the to-be-produced tooth and the scaling of the alternative dental crown, in order to ensure the best size matching of the alternative dental crown;

5.2) occlusal surface adjustment: adjusting the normal vector of the dental surface of the alternative dental crown according to the maxillofacial feature vector of the opposite jaw tooth of the to-be-produced tooth to best fit;

5.3) adjusting the lower edge of the dental crown: adjusting the coordinates of each point on the lower edge of the alternative dental crown according to the distance from each point on the abutment edge line to the occlusal plane calculated in step 3.3), so that the distance from each point on the lower edge of the alternative dental crown to the highest point on the occlusal surface matches the distance from each point on the abutment edge line to the occlusal plane.

8. Application of the intelligent design method of a digital model for an oral digital impression instrument according to any of claims 1-7 in chair side design.

## Patentansprüche

1. Intelligentes Entwurfsverfahren für ein digitales Modell für ein digitales Abdruckgerät für die Mundhöhle, wobei das Verfahren umfasst:

1) Einrichtung einer Datenbank: Daten über alternative Zahnkronen, die in einer XML-Datei gespeichert sind. Mindestens eine der gespeicherten Informationen über alternative Zahnkronendaten, einschließlich einer Zahnnummer, eines Zahnkronentyps, einer Gesamtgrößen-Merkmalsvektors einer Zahnkrone und eines Speicherpfads eines Zahns,

Der Gesamtgrößen-Merkmalsvektor der Zahnkrone ist *(Größe_x, Größe_y, Größe_z)*, der durch Berechnung mit einem Sechs-Punkte-Verfahren erhalten wird, und das Sechs-Punkte- Verfahren besteht darin, sechs Umriss-Hochpunkte durch die maximalen und minimalen Koordinaten der Zahnkrone zu erhalten, von denen die beiden Umriss-Hochpunkte der mesialen und distalen Flächen sind $P^x_{max}$ und $P^x_{min}$, die beiden Umriss-Hochpunkte der bukkalen und lingualen Flächen sind $P^y_{max}$ und $P^y_{min}$ und die beiden Umriss-Hochpunkte der Umrisse der unteren und oberen Flächen sind $P^z_{max}$ und $P^z_{min}$. Die *Größe_x* ist der Absolutwert der Differenz zwischen den x-Koordinaten der Umriss-Hochpunkte $P^x_{max}$ und $P^x_{min}$. Die *Größe_y* ist der Absolutwert der Differenz zwischen den y-Koordinaten der Umriss-Hochpunkte $P^y_{max}$ und $P^y_{min}$, und die *Größe_z* ist der Absolutwert der Differenz zwischen den z-Koordinaten der Umriss-Hochpunkte $P^z_{max}$ and $P^z_{min}$;

2) Datenerfassung: Erfassung von 3D-Modelldaten eines Mundhöhlenmodells nach der Zahnpräparation mit einem oralen digitalen Erfassungsgerät. Der von dem oralen digitalen Erfassungsgerät erfassten Daten, einschließlich eines Oberkiefers und eines Unterkiefers, sowie Bestätigung der Kaubeziehungen zwischen dem Oberkiefer und dem Unterkiefer;

3) Vorverarbeitung der Daten: Beschriftung des in Schritt 2) erhaltenen 3D-Modells der Mundhöhle und Beschriftung einer Pfeilerzahnposition, des Zahnkronentyps und einer Pfeiler-Schneidkante;

4) Intelligentes Design: Durchführung der automatischen Designverarbeitung an einem herzustellenden Zahn gemäß dem 3D-Modell, das durch die Vorverarbeitung in Schritt 3) erhalten wurde, und Abgleich einer der alternativen Zahnkronen aus der Datenbank;

5) Abgleich alternativer Zahnkronen: Bestimmung der Ähnlichkeit zwischen einer alternativen Zahnkrone und einem herzustellenden Zahn gemäß dem Kosinus eines eingeschlossenen Winkels zwischen dem Gesamtgrößen-Merkmalsvektor der Zahnkrone der alternativen Zahnkrone und dem Gesamtgrößen-Merkmalsvektor der Zahnkrone des herzustellenden Zahns, wobei der Kosinus des eingeschlossenen Winkels [0,1] beträgt;

Abgleich alternativer Zahnkronen in Schritt 5) umfasst weiterhin:

4.1) Abgleich von Zahnnummer und Zahnkronentyp: Ermittlung von alternativen Zahnkronendaten in der Datenbank entsprechend der Zahnnummer des ausgewählten Pfeilers und des ausgewählten Zahnkronentyps (Einzelkrone oder Kronenbrücke);

4.2) Abgleich der Gesamtgröße: Berechnung des Gesamtgrößen-Merkmalsvektors der Zahnkrone des herzustellenden Zahns als ($Größe\_ x_0$, $Größe\_ y_0$, $Größe\_ z_0$) und des Gesamtgrößen-Merkmalsvektors der Zahnkrone der alternativen Zahnkrone als (*Größe\_ x'*, *Größe\_ y'*, *Größe\_ z'*), und Bestimmen der Ähnlichkeit zwischen der alternativen Zahnkrone und dem herzustellenden Zahn gemäß dem Kosinus zwischen dem Gesamtgrößen-Merkmalsvektor der Zahnkrone der alternativen Zahnkrone und dem Gesamtgrößen-Merkmalsvektor der Zahnkrone des herzustellenden Zahns. Je größer der Kosinus des eingeschlossenen Winkels ist, desto kleiner ist der eingeschlossene Winkel der beiden Vektoren. Wenn die Richtungen der beiden Vektoren zusammenfallen, ist der maximale Kosinus des eingeschlossenen Winkels 1;

4.3) Abgleich des lokalen Merkmals: Berechnung des komplementären Vektors ($f1'$, $f2'$,..., $fn'$) des Kauflächen-Merkmalsvektors ($f1$, $f2$,..., $fn$) des gegenüberliegenden Kieferzahns des herzustellenden Zahns, wobei der komplementäre Vektor ($f1'$, $f2'$,..., $fn'$) = (- $f1$,- $f2$,..., -$fn$) , und Durchführung eines lokalen Merkmalsabgleichs durch Berechnung der Kosinus-Ähnlichkeit des komplementären Vektors ($f1'$, $f2'$,..., $fn'$) □und des maxillofazialen Merkmalsvektors ($F1, F2,..., Fn$) der alternativen Zahnkrone;

6) Anpassung des Zahnmusters: Anpassung entsprechend einer Skalierung des Gesamtgrößen-Merkmalsvektors des herzustellenden Zahns, eines maxillofazialen Merkmalsvektors und der alternativen Zahnkrone;
7) Datenausgabe: Das entworfene Zahnmodell wird in einem Datenformat exportiert, das für den Import durch 3shape und EXOcad Software angepasst ist.

2. Intelligentes Entwurfsverfahren nach Anspruch 1, wobei die Datenerfassung in Schritt 2) außerdem umfasst:

1.1) Erfassen von 3D-Modelldaten des Oberkiefers und des Unterkiefers bzw. bukkalen Daten bei der Okklusion;
1.2) Berechnung und Fixierung des Kontaktverhältnisses zwischen den 3D-Modellen des Oberkiefers und des Unterkiefers durch bukkale Daten oder manuelle Verschiebung und Drehung der 3D-Modelle des Oberkiefers und des Unterkiefers im 3D-Koordinatensystem, so dass die 3D-Modelle des Oberkiefers und des Unterkiefers in einer korrekten Kaubeziehung stehen.

3. Intelligentes Entwurfsverfahren nach Anspruch 1, wobei die Datenerfassung in Schritt 3) außerdem umfasst:

2.1) Auswahl des Pfeilers nach der Zahnpräparation, Hervorhebung und Aufzeichnung der Zahnnummer der Zahnposition, wobei die Zahnnummernregel der Zahnposition durch die FDI-Notation angegeben wird;
2.2) Auswahl des Zahnkronentyps des Pfeilers als Einzelkrone oder Kronenbrücke: bei einer Einzelkrone Aufzeichnung der Zahnnummer des Pfeilers. Bei einer Kronenbrücke Aufzeichnung der Zahnnummern des äußersten linken und des äußersten rechten Pfeilers der Kronenbrücke und der Zahnnummern aller herzustellenden Zähne;
2.3) Interaktives Markieren der Schneidkanten: Auswählen der Punkte des Dreiecksnetzes auf der Schneidkante des Pfeilers, Skizzieren der Punkte der Pfeiler- Schneidkante nacheinander und Abschließen der Auswahl der Pfeiler - Schneidkante. Hervorheben der letzten Schneidkante mit einer speziellen Farbe.

4. Intelligentes Entwurfsverfahren nach Anspruch 1, wobei das Entwurfsverfahren in Schritt 4) außerdem umfasst:

3.1) Berechnung des Abstands zwischen benachbarten Zähnen: interaktives Auswählen der beiden Punkte, die auf der Schneidkante des Zahnpfeilers den benachbarten Zähnen am nächsten liegen, und Berechnung des Abstands zwischen diesen beiden Punkten, um den Abstand zwischen benachbarten Zähnen zu ermitteln;
3.2) Berechnung der bukkalen und lingualen Konvexitätskurven des Zahnbogens: Anpassung der Konvexitätskurve des Zahnbogens nach der auf der Funktion β basierende Methode gemäß den in Schritt 2 erhaltenen 3D-Modelldaten des Oberkiefers und des Unterkiefers);
3.3) Berechnung der Höhe des okklusalen Zahnfleischdurchmessers: Berechnung des Abstands von jedem Punkt auf der Schneidkante des Zahnpfeilers zur Kauebene entsprechend der gewählten Schneidkante des Zahnpfeilers und Berechnung des durchschnittlichen Abstands als Höhe des okklusalen Zahnfleischdurchmessers;
3.4) Berechnung der Höckergruben- und Fissuren-Brücke-Formmerkmale der gegenüberliegenden Kiefer: Berechnung der Kauflächenmerkmale der gegenüberliegenden Kieferzähne gemäß den in Schritt 2) erhaltenen 3D-Modelldaten des Ober- und Unterkiefers.

5. Intelligentes Entwurfsverfahren nach Anspruch 4, wobei Schritt 3.2) außerdem umfasst:

3.2.1) Ermittlung des bukkalen Apex der Schneidezahn-Kontaktfläche des Kiefers, an dem sich der Pfeiler befindet, des Punktes der bilateralen Eckzähne mit der maximalen bukkalen Krümmung und des Punktes der bilateralen zweiten bleibenden Backenzähne mit der maximalen bukkalen Krümmung;

3.2.2) Bestimmung β Funktion gemäß den fünf Punkten in Schritt 3.2.1) zur Anpassung an die Konvexitätskurve des Zahnbogens;

3.2.3) Wiederholen der Schritte 3.2.1) und 3.2.2) und Berechnung der Konvexitätskurve des lingualen Zahnbogens auf die gleiche Weise.

**6.** Intelligentes Entwurfsverfahren nach Anspruch 4, wobei Schritt 3.4) außerdem umfasst:

3.4.1) Interaktives Auswählen der Kaufläche des gegenüberliegenden Kieferzahns;

3.4.2) Berechnung des Merkmalsvektors der ausgewählten Kaufläche: Zunächst wird die effektive Nachbarschaft um die Höckergrube und die Fissuren-Brücke in der Kaufläche gemäß den Auswahlergebnissen ermittelt, dann wird ein lokales sphärisches Koordinatensystem für die Fläche erstellt, anschließend werden die Elevation und der Azimut der Normalen an jedem Scheitelpunkt der Fläche durch die statistische 2D-Histogramm-Methode berechnet und der Positionsindex bestimmt. Schließlich wird der maxillofaziale Merkmalsvektor ($f1$, f2,..., $fn$) des gegenüberliegenden Kieferzahns entsprechend dem 2D-Histogramm.

**7.** Intelligentes Designverfahren nach Anspruch 4, wobei die Zahnmusteranpassung in Schritt 6) außerdem umfasst:

5.1) Integrale Anpassung: Anpassung mit Hilfe der Skalierungsmatrix S entsprechend dem Gesamtgrößen-Merkmalsvektor des herzustellenden Zahns und der Skalierung der alternativen Zahnkrone, um die beste Größenanpassung der alternativen Zahnkrone zu gewährleisten;

5.2) Anpassung der okklusalen Oberfläche: Anpassung des Normalvektors der Zahnoberfläche der alternativen Zahnkrone entsprechend der maxillofazialen Merkmalsvektor des gegenüberliegenden Kieferzahns des herzustellenden Zahns, um ihn optimal anzupassen;

5.3) Anpassen der Unterkante der Zahnkrone: Anpassung der Koordinaten jedes Punktes auf der Unterkante der alternativen Zahnkrone entsprechend dem in Schritt 3.3) berechneten Abstand von jedem Punkt auf der Pfeiler- Schneidkante zur Kauebene, so dass der Abstand von jedem Punkt auf der Unterkante der alternativen Zahnkrone zum höchsten Punkt auf der Kaufläche dem Abstand von jedem Punkt auf der Pfeiler- Schneidkante zur Kauebene entspricht.

**8.** Anwendung des intelligenten Entwurfsverfahrens eines digitalen Modells für ein digitales Abdruckgerät für die Mundhöhle nach einem der Ansprüche 1 bis 7 in dem stuhlseitigen Design.

## Revendications

**1.** Méthode de conception intelligente d'un modèle digital pour un instrument de prise d'empreinte orale digitale, la méthode comprenant :

1) établissement de la base de données : les données alternatives de couronne dentaire étant stockées dans un fichier XML, au moins une des informations de données alternatives de couronne dentaire stockées comprenant un numéro de dent, un type de couronne dentaire, un vecteur de caractéristiques de taille globale d'une couronne dentaire et un chemin de stockage d'une dent ;

le vecteur de caractéristiques de taille globale de la couronne dentaire est *(taille _ x, taille _ y, taille _ z)*, qui est obtenu par calcul par une méthode à six points, et la méthode à six points consiste à obtenir six points hauts de contour par les coordonnées maximales et minimales de la couronne dentaire, dont les deux points hauts de contour des surfaces mésiale et distale sont $P^x_{min}$ et $P^x_{max}$, les deux points hauts de contour des surfaces buccale et linguale sont $P^y_{min}$ et $P^y_{max}$, et les deux points hauts de contour des surfaces inférieure et supérieure sont $P^z_{min}$ et $P^z_{max}$ ; *taille _ x* est la valeur absolue de la différence entre les coordonnées x des points hauts de contour $P^x_{min}$ et $P^x_{max}$, *taille _ y* est la valeur absolue de la différence entre les coordonnées y des points hauts de contour $P^y_{min}$ et $P^y_{max}$, et *taille _ z* est la valeur absolue de la différence entre les coordonnées z des points hauts de contour $P^z_{min}$ et $P^z_{max}$;

2) acquisition de données : acquisition des données du modèle 3D d'une cavité orale après préparation d'une dent avec un équipement d'acquisition digitale orale, les données acquises par l'équipement d'acquisition digitale orale comprenant une mâchoire supérieure et une mâchoire inférieure, ainsi que la confirmation des relations d'occlusion entre la mâchoire supérieure et la mâchoire inférieure ;

3) prétraitement des données : marquage du modèle 3D de la cavité orale obtenu à l'étape 2), et marquage d'une position de dent pilier, du type de couronne dentaire et d'une ligne de bord de pilier ;

4) conception intelligente : réalisation du traitement de conception automatique sur une dent à produire selon le modèle 3D obtenu par le prétraitement à l'étape 3), et correspondance d'une des couronnes dentaires alternatives de la base de données ;

5) correspondance de la couronne dentaire alternative : détermination de la similarité entre une couronne dentaire alternative et une dent à produire selon le cosinus d'un angle inclus entre le vecteur de caractéristiques de taille globale de la couronne dentaire de la couronne dentaire alternative et le vecteur de caractéristiques de taille globale de la couronne dentaire de la dent à produire, le cosinus de l'angle inclus étant [0,1] ;

la correspondance de la couronne dentaire alternative à l'étape 5) comprend en outre :

4.1) correspondance du numéro de dent et du type de couronne dentaire : recherche des données alternatives de couronne dentaire dans la base de données selon le numéro de dent du pilier sélectionné et le type de couronne dentaire sélectionné comme couronne simple ou bridge ;

4.2) correspondance de taille globale : calcul du vecteur de caractéristiques de taille globale de la couronne dentaire de la dent à produire comme *(taille _ $x_0$, taille _ $y_0$, taille _ $z_0$)* et du vecteur de caractéristiques de taille globale de la couronne dentaire de la couronne dentaire alternative comme *(taille _ x', taille _ y', taille _ z')*, et détermination de la similarité entre la couronne dentaire alternative et la dent à produire selon le cosinus entre le vecteur de caractéristiques de taille globale de la couronne dentaire de la couronne dentaire alternative et le vecteur de caractéristiques de taille globale de la couronne dentaire de la dent à produire ; plus le cosinus de l'angle inclus est grand, plus l'angle inclus des deux vecteurs est petit ; lorsque les directions des deux vecteurs coïncident, le cosinus maximum de l'angle inclus est 1 ;

4.3) correspondance des caractéristiques locales : calcul du vecteur complémentaire *(f1', f2',..., fn')* du vecteur de caractéristiques de la surface occlusale *(f1, f2,..., fn)* de la dent opposée de la dent à produire, le vecteur complémentaire *(f1', f2',..., fn') = (- f1, - f2,..., - fn)*, et réalisation de la correspondance des caractéristiques locales en calculant la similarité du cosinus du vecteur complémentaire *(f1', f2',..., fn')* et du vecteur de caractéristiques maxillaires *(F1, F2,..., Fn)* de la couronne dentaire alternative ;

6) ajustement du modèle dentaire : ajustement selon un redimensionnement du vecteur de caractéristiques de taille globale de la dent à produire, un vecteur de caractéristiques maxillaires et la couronne dentaire alternative ;

7) sortie des données : le modèle de dent conçu étant exporté sous un format de données adapté à être importé par les logiciels 3shape et EXOcad.

2. La méthode de conception intelligente selon la revendication 1, dans lequel l'acquisition de données à l'étape 2) comprend en outre :

1.1) acquisition des données du modèle 3D de la mâchoire supérieure et de la mâchoire inférieure et des données buccales à l'occlusion respectivement ;

1.2) calcul et fixation de la relation de contact entre les modèles 3D de la mâchoire supérieure et de la mâchoire inférieure par des données buccales, ou traduction et rotation manuelles des modèles 3D de la mâchoire supérieure et de la mâchoire inférieure dans le système de coordonnées 3D, de sorte que les modèles 3D de la mâchoire supérieure et de la mâchoire inférieure soient dans une relation d'occlusion correcte.

3. La méthode de conception intelligente selon la revendication 1, dans lequel le prétraitement des données à l'étape 3) comprend en outre :

2.1) sélection du pilier après préparation de la dent, le mettre en évidence, et enregistrement du numéro de dent de la position de la dent, la règle du numéro de dent de la position de la dent étant donnée par la notation FDI ;

2.2) sélection du type de couronne dentaire du pilier comme couronne simple ou bridge : s'il s'agit d'une couronne simple, enregistrement du numéro de dent du pilier ; s'il s'agit d'un bridge, enregistrement des numéros de dent des piliers les plus à gauche et à droite du bridge et des numéros de dent de toutes les dents à produire ;

2.3) marquage des lignes de bord de manière interactive : sélection des points de maillage triangulaire sur la ligne de bord du pilier, esquisse des points de la ligne de bord du pilier un par un, et achèvement de la sélection de la ligne de bord du pilier ; mise en évidence de la ligne de bord finale avec une couleur spéciale.

4. La méthode de conception intelligente selon la revendication 1, dans lequel la conception intelligente à l'étape 4) comprend en outre :

3.1) calcul de la distance entre les dents adjacentes : sélection interactive des deux points les plus proches des dents adjacentes sur la ligne de bord du pilier, et calcul de la distance entre ces deux points pour obtenir la distance entre les dents adjacentes ;

3.2) calcul des courbes de convexité de l'arc dentaire buccal et lingual : ajustement de la courbe de convexité de l'arc dentaire par la méthode basée sur la fonction β selon les données du modèle 3D de la mâchoire supérieure et de la mâchoire inférieure obtenues à l'étape 2) ;

3.3) calcul de la hauteur du diamètre de la gencive occlusale : calcul de la distance de chaque point de la ligne de bord du pilier au plan occlusal selon la ligne de bord du pilier sélectionnée, et calcul de la distance moyenne comme hauteur du diamètre de la gencive occlusale ;

3.4) calcul des caractéristiques de forme des fosses et crêtes de la surface occlusale des mâchoires opposées : calcul des caractéristiques de la surface occlusale des dents opposées selon les données du modèle 3D de la mâchoire supérieure et de la mâchoire inférieure obtenues à l'étape 2).

**5.** La méthode de conception intelligente selon la revendication 4, dans lequel l'étape 3.2) comprend en outre :

3.2.1) obtention de l'apex buccal de la surface de contact de l'incisive de la mâchoire où se trouve le pilier, du point des canines bilatérales avec la courbure buccale maximale, et du point des deuxièmes molaires permanentes bilatérales avec la courbure buccale maximale ;

3.2.2) détermination de la fonction β selon les cinq points obtenus à l'étape 3.2.1) pour ajuster la courbe de convexité de l'arc dentaire ;

3.2.3) répétition des étapes 3.2.1) et 3.2.2), et calcul de la courbe de convexité de l'arc dentaire lingual de la même manière.

**6.** La méthode de conception intelligente selon la revendication 4, dans lequel l'étape 3.4) comprend en outre :

3.4.1) sélection interactive de la surface occlusale de la dent opposée ;

3.4.2) calcul du vecteur de caractéristiques de la surface occlusale sélectionnée : d'abord, obtention du voisinage effectif autour de la fosse et de la crête de la surface occlusale selon les résultats de la sélection, ensuite, établissement d'un système de coordonnées sphériques locales pour la surface, puis, calcul de l'élévation et de l'azimut de la normale à chaque sommet de la surface par la méthode statistique de l'histogramme 2D, et détermination de l'indice de position à tour de rôle ; enfin, génération du vecteur de caractéristiques maxillaires ($f1, f2,..., fn$) de la dent opposée selon l'histogramme 2D.

**7.** La méthode de conception intelligente selon la revendication 4, dans lequel l'ajustement du modèle dentaire à l'étape 6) comprend en outre

5.1) ajustement intégral : ajustement par la matrice de mise à l'échelle S selon le vecteur de caractéristiques de taille globale de la dent à produire et la mise à l'échelle de la couronne dentaire alternative, afin d'assurer la meilleure correspondance de taille de la couronne dentaire alternative ;

5.2) ajustement de la surface occlusale : ajustement du vecteur normal de la surface dentaire de la couronne dentaire alternative selon le vecteur de caractéristiques maxillaires de la dent opposée de la dent à produire pour un meilleur ajustement ;

5.3) ajustement du bord inférieur de la couronne dentaire : ajustement des coordonnées de chaque point sur le bord inférieur de la couronne dentaire alternative selon la distance de chaque point de la ligne de bord du pilier au plan occlusal calculée à l'étape 3.3), de sorte que la distance de chaque point sur le bord inférieur de la couronne dentaire alternative au point le plus élevé de la surface occlusale corresponde à la distance de chaque point de la ligne de bord du pilier au plan occlusal.

**8.** Application de la méthode de conception intelligente d'un modèle digital pour un instrument de prise d'empreinte orale digitale selon l'une quelconque des revendications 1 à 7 dans la conception à l'accotoir de fauteuil.

Fig. 1

Fig. 2

(0,D)

Right canine

Left canine point (X2, Y2)

(−W/2,0)

Origin (0, 0)

(−W/2,0)

Fig. 3

$P_{max}^{y}$

$P_{max}^{z}$

$P_{min}^{x}$

$P_{max}^{x}$

$P_{min}^{y}$

$P_{min}^{z}$

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000107203 A
- US 2015073577 A1
- EP 2422740 A1